# EUROPEAN PATENT SPECIFICATION

(11) **EP 2 770 858 B1**
(45) Date of publication and mention of the grant of the patent: **02.08.2017**
(21) Application number: 12790442.3
(22) Date of filing: 24.10.2012
(51) Int. Cl.: A24F 47/00

(54) **AEROSOL GENERATING DEVICE WITH HEATER ASSEMBLY**
AEROSOLERZEUGUNGSVORRICHTUNG MIT HEIZERANORDNUNG
DISPOSITIF GÉNÉRATEUR D'AÉROSOL DOTÉ D'UN ENSEMBLE FORMANT CHAUFFAGE

(30) Priority: 25.10.2011 EP 11250870
(43) Date of publication of application: 03.09.2014
(73) Proprietor: Philip Morris Products S.A., 2000 Neuchâtel (CH)
(72) Inventor: RUSCIO, Dani, 2088 Cressier (CH); GREIM, Olivier, 1423 Villars-Burquin (CH); PLOJOUX, Julien, 1208 Geneva (CH)
(74) Representative: Ponder, William Anthony John
(86) International application number: PCT/EP2012/071083
(87) International publication number: WO 2013/060743

(56) References cited:
- EP-A1- 2 327 318
- EP-A1- 2 340 730
- EP-A2- 0 430 559
- US-A- 5 269 327
- US-A- 5 591 368
- US-A- 5 934 289
- US-A1- 2005 045 193

## Description

The present invention relates to an aerosol generating device with an improved heater assembly. The invention finds particular application in an electrically operated smoking system as a heater assembly for heating an aerosol-forming substrate.

Patent application US-A-5,269,327 discloses an electrical smoking article comprising a plurality of charges of tobacco flavor medium which are heated sequentially to provide individual puffs. The sequential heating can be provided either by a plurality of individually activated heater elements or portions of a heater element, or by a single movable heater element.

It would be advantageous to provide an aerosol generating system that improves on these known schemes for heating an aerosol-forming substrate by providing a more developed, compact and efficient solution.

According to one aspect of the invention there is provided an aerosol generating device, comprising: a housing configured to receive an aerosol-forming substrate having an internal cavity; a heating element, the heating element configured to be received within the internal cavity of the aerosol-forming substrate; and a positioning mechanism coupled to the heating element, the positioning mechanism configured to move the heating element between a plurality of heating positions within the internal cavity.

The use of a movable heating element within the aerosol-forming substrate means that efficient heating can be accomplished with minimal heat losses. The use of a heating element within the aerosol-forming substrate also means that the external diameter of the device can be minimized because insulation is not necessary as compared to externally positioned heater elements. The distance that the heating element is required to move within the aerosol-forming substrate can be made very small, and is smaller than would be required to move between corresponding portions of the substrate if the heating element were positioned externally of the aerosol-forming substrate.

The positioning mechanism may be coupled to the housing. Preferably, the positioning mechanism further comprises an engagement mechanism. The engagement mechanism is configured to move the heating element towards or away from an interior surface of the internal cavity. More preferably, the engagement mechanism is configured to move the heating element into or out of contact with the interior surfaces of the aerosol-forming substrate. This allows for efficient thermal transfer from the heating element to the aerosol-forming substrate during heating. The engagement mechanism also allows for smooth movement between heating positions when the heating element is not activated, and for easy insertion and removal of the aerosol-forming substrate.

The heating element may be placed in direct thermal contact with the aerosol-forming substrate or may be positioned close to the substrate without contacting it. Alternatively, the heating element may be in indirect contact with the aerosol-forming substrate. For example, a conductive layer may be provided between the heating element and the aerosol-forming substrate. The conductive layer may be a foil layer that conducts heat from the heating element to the aerosol-forming substrate but prevents damage to the aerosol-forming substrate caused by movement of the heating element. It may also spread the heat contact area or contact force so as not to damage the aerosol-forming substrate.

Advantageously, the heating element is heated electrically. However, it is possible to use other heating schemes to heat the heating element, for example by heat conduction from another heat source or by magnetic induction heating of the heating element.

The internal cavity of the aerosol-forming substrate can be of any size and shape provided that the heating element and any necessary parts of the positioning mechanism can be received within it. Preferably, the device is configured to receive an aerosol-forming substrate that is substantially tubular. Preferably, the tubular aerosol-forming substrate defines a hole extending through at least a portion of its length and the positioning mechanism is configured to move the heating element to different positions along this portion of the length of the aerosol-forming substrate.

It could be desirable for electrically heated smoking devices to mimic as far as possible a conventional lit-end combustible cigarette. By moving the heating element longitudinally (that is, along the length of the device) to heat different sections of the aerosol-forming substrate within the device, a large number of individual puffs can be achieved without impacting the diameter of the device. Accordingly, the device can be made to closely mimic the shape of a conventional lit-end combustible cigarette. Moving the heater circumferentially to heat separate sections of the aerosol-forming substrate is an alternative option in accordance with the present invention.

Preferably, the heating element is substantially ring shaped or circular. This is particularly advantageous when the heating element is configured to move along the length of a tubular aerosol-forming substrate. A ring shaped or circular heating element can efficiently heat a corresponding ring shaped or circular section of the aerosol-forming substrate.

Preferably, the heating element is resilient, and so returns to its original shape after being bent, compressed or stretched. Preferably, the engagement mechanism is attached to at least one end of the heating element. The engagement mechanism is configured to move the at least one end of the heating element in order to radially expand or contract the heating element. The engagement mechanism may comprise a radial arm attached to the end of the heating element and configured to pivot about a longitudinal axis. In this way the radial arm is moved to expand or contract the heating element.

The positioning mechanism may be mechanically activated or electronically activated. The device may include a microcontroller. The microcontroller may be configured to control the supply of electrical power to the heating element. In addition, the microcontroller may be configured to activate the positioning mechanism following the supply of predetermined amount or duration of electrical power to the heating element.

The positioning mechanism may be entirely mechanical and rely on a user to manually move an element on the device to operate the positioning mechanism. Alternatively, the positioning mechanism may be driven automatically using an electrically powered mechanism, such as an electromagnetic, electrostatic or piezoelectric actuation mechanism. Alternatively, the positioning mechanism may be driven by a combination of mechanical and electrical mechanisms. The positioning mechanism may also include a ratchet or other means to ensure that the same portion of the aerosol-forming substrate cannot be heated twice.

The heating element requires a supply of energy. Preferably, the positioning mechanism is configured to conduct electricity to the heating element. The positioning mechanism therefore preferably comprises low resistivity, but mechanically rigid materials, such as copper. However, separate wiring may alternatively be provided to supply electrical energy to the heating element.

Preferably, the heating element has a comparatively higher resistivity in order to give rise to a significant Joule heating effect, and may be formed from a various materials, including materials such as a Nichrome™ alloy or Titanium alloy and others which may have similar properties. The heating element typically has a resistivity ranging between 140 and 170 micro-ohm per centimeter. As previously described, it also desirable for the heating element to be resilient and to have suitable mechanical strength so as to provide a robust and reliable heating arrangement over many cycles of use. This is an important factor when choosing a material giving the required resistance.

The device may comprise a plurality of heating elements. The device may comprise two, three, four or more heating elements. The positioning mechanism may be configured to move some or all of the heating elements. The device may include a first heating element configured to be received within an internal cavity of the aerosol-forming substrate and a second heating element configured to be positioned externally of the aerosol forming substrate. The positioning mechanism may be configured to move the first and second heating elements in a coordinated fashion so that the first and second heating elements are positioned to simultaneously heat the same or different portions of the aerosol-forming substrate.

The operating or working temperature of the heating element may be approximately 50°C to 500°C. Depending on the aerosol forming substrate, preferably the operating temperature or working temperature of the heating element may be 50°C to 100°C. For other aerosol forming substrates, preferably the operating temperature or working temperature of the heating element may be 250°C to 300°C. The temperature must be sufficiently high to form an aerosol with desired droplet size while significantly reducing the risk of, or avoiding, pyrolysis and combustion. The operating temperature range needed to form an aerosol and avoid pyrolysis and combustion is dependent on the different aerosol forming substrate components, their combinations and the contact surface of the heater configuration. However, a temperature range between 50 °C and 500 °C would be adequate to form an aerosol while significantly reducing the risk of, or avoiding, pyrolysis and combustion.

As is known to those skilled in the art, an aerosol is a suspension of solid particles or liquid droplets in a gas, such as air. The aerosol-forming substrate preferably comprises a tobacco-containing material containing volatile tobacco flavour compounds which are released from the aerosol-forming substrate upon heating. Alternatively or in addition, the aerosol-forming substrate may comprise a non-tobacco material. Preferably, the aerosol-forming substrate further comprises a suitable aerosol former. The aerosol former may be any suitable known compound or mixture of compounds that, in use, facilitates formation of a dense and stable aerosol and that is substantially resistant to thermal degradation at the operating temperature. Suitable aerosol formers are well known in the art and include, for example, polyhydric alcohols, esters of polyhydric alcohols, such as glycerol mono-, di- or triacetate, and aliphatic esters of mono-, di- or polycarboxylic acids, such as dimethyl dodecanedioate and dimethyl tetradecanedioate. Preferred aerosol formers for use in smoking articles according to the invention are polyhydric alcohols or mixtures thereof, such as triethylene glycol, 1,3-butanediol and, most preferred, glycerine. Another suitable aerosol former is propylene glycol.

The aerosol-forming substrate is preferably a solid aerosol-forming substrate. The solid aerosol-forming substrate may comprise, for example, one or more of: powder, granules, pellets, shreds, spaghettis, strips or sheets containing one or more of: herb leaf, tobacco leaf, fragments of tobacco ribs, reconstituted tobacco, homogenised tobacco, extruded tobacco and expanded tobacco. The solid aerosol-forming substrate may be in loose form, or may be provided in a suitable container or cartridge. Optionally, the solid aerosol-forming substrate may contain additional tobacco or non-tobacco volatile flavour compounds, to be released upon heating of the aerosol-forming substrate.

Optionally, the solid aerosol-forming substrate may be provided on or embedded in a thermally stable carrier. In a preferred embodiment, the carrier is a tubular carrier having a thin layer of the solid aerosol-forming substrate deposited on its inner surface, or on its outer surface, or on both its inner and outer surfaces. Such a tubular carrier may be formed of, for example, a paper, or paper like material, a non-woven carbon fibre mat, a low mass open mesh metallic screen, or a perforated metallic foil or any other thermally stable polymer matrix. Alternatively, the carrier may take the form of powder, granules, pellets, shreds, spaghettis, strips or sheets.

The solid aerosol-forming substrate may be deposited on the surface of the carrier in the form of, for example, a sheet, foam, gel or slurry. The solid aerosol-forming substrate may be deposited on the entire surface of the carrier, or alternatively, may be deposited in a pattern in order to provide a non-uniform flavour delivery during use.

Alternatively, the carrier may be a non-woven fabric or fibre bundle into which tobacco components have been incorporated. The non-woven fabric or fibre bundle may comprise, for example, carbon fibres, natural cellulose fibres, or cellulose derivative fibres.

Alternatively, the aerosol-forming substrate may be a liquid aerosol-forming substrate. If a liquid aerosol-forming substrate is provided, the electrically heated smoking system preferably comprises means for retaining the liquid. For example, the liquid aerosol-forming substrate may be retained in a container. Alternatively or in addition, the liquid aerosol-forming substrate may be absorbed into a porous carrier material. The porous carrier material may be made from any suitable absorbent plug or body, for example, a foamed metal or plastics material, polypropylene, terylene, nylon fibres or ceramic. The liquid aerosol-forming substrate may be retained in the porous carrier material prior to use of the electrically heated smoking system or alternatively, the liquid aerosol-forming substrate material may be released into the porous carrier material during, or immediately prior to use. For example, the liquid aerosol-forming substrate may be provided in a capsule. The shell of the capsule preferably melts upon heating and releases the liquid aerosol-forming substrate into the porous carrier material. The capsule may optionally contain a solid in combination with the liquid.

During operation, the aerosol-forming substrate may be completely contained within the aerosol generating device. In that case, a user may puff on a mouthpiece of the electrically heated smoking system. Alternatively, during operation, the aerosol-forming substrate may be partially contained within the electrically heated smoking device. In that case, the aerosol-forming substrate may form part of a separate article and the user may puff directly on the separate article.

The electrically heated smoking device may further comprise a sensor to detect air flow indicative of a user taking a puff. In that embodiment, preferably, the sensor is connected to the power supply and the system is arranged to energise the at least one heater when the sensor detects a user taking a puff. Alternatively, the system may further comprise a manually operable switch, for a user to initiate a puff.

The device may include a power supply. In one preferred embodiment, the power supply is a DC voltage source. In one embodiment, the power supply is a Lithium-ion battery. Alternatively, the power supply may be a Nickel-metal hydride battery, a Nickel cadmium battery or a Lithium Phosphate battery.

The electrically heated smoking device may further comprise an interface. The interface may alternatively or additionally facilitate other functionality and features for the smoking system. For that purpose, the connection may be a wired connection (such as a USB connection) or a wireless connection (such as a Bluetooth connection). Preferably, the interface facilitates bi-directional communication between the secondary unit and an intelligent device or host that has its own computing capability and is capable of acting as the primary power supply. This may allow data to be downloaded from the intelligent device or host to the secondary unit and data to be uploaded from the secondary unit to the intelligent device or host.

An embodiment of the invention will further be described, by way of example only, with reference to the accompanying drawings, in which:
Figure 1 is a schematic view of the basic elements of an aerosol-generating device in accordance with the invention;
Figure 2 is a schematic representation of an aerosol-generating device according to one embodiment of the invention;
Figure 3 is schematic cross section of the device of Figure 2;
Figure 4 is a schematic representation of an aerosol-generating device according to another embodiment of the invention;
Figure 5 is a schematic cross-section of the device of Figure 4;
Figure 6 is a perspective view of a heater assembly in accordance with the embodiment of Figure 2; and
Figure 7 is a flow diagram illustrating the steps taken during the operation of the device of Figure 2.

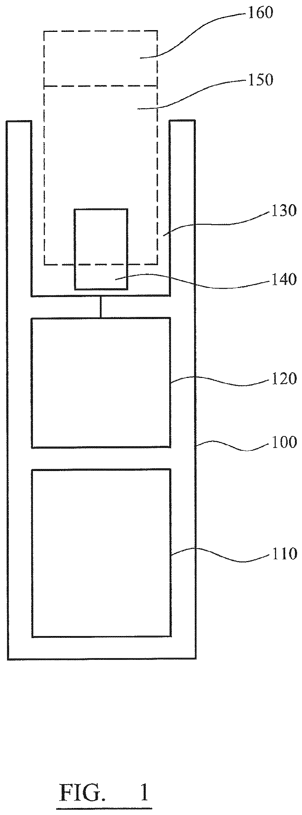

Figure 1 is a schematic illustration of an electrically powered smoking device. The device comprises a housing 100 containing a battery 110, control electronics 120 and a heater 140, together with a positioning mechanism for moving the heater. The heater 140 is positioned within a socket 130 configured to receive a consumable element 150 containing an aerosol-forming substrate. The consumable element also comprises a filter element 160 through which a user inhales aerosol formed in the device. The aerosol-forming substrate is heated by the heater and releases vaporized flavor compounds. The vapors nucleate to form an aerosol, which is drawn through the filter 160 by a user inhalation.

The heater is configured and controlled to provide short bursts of heat on a per-puff basis. The heater heats a new portion of the aerosol-forming substrate for each puff, to ensure that the desired amount and desired characteristics of aerosol are achieved.

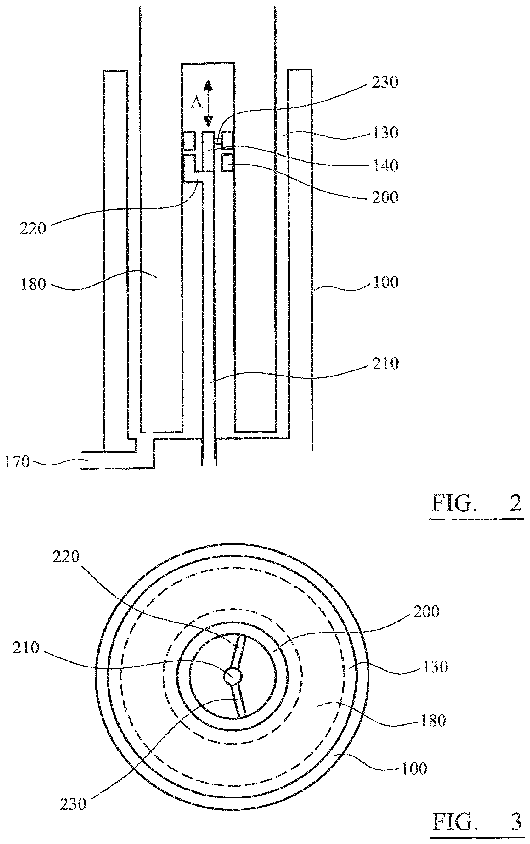

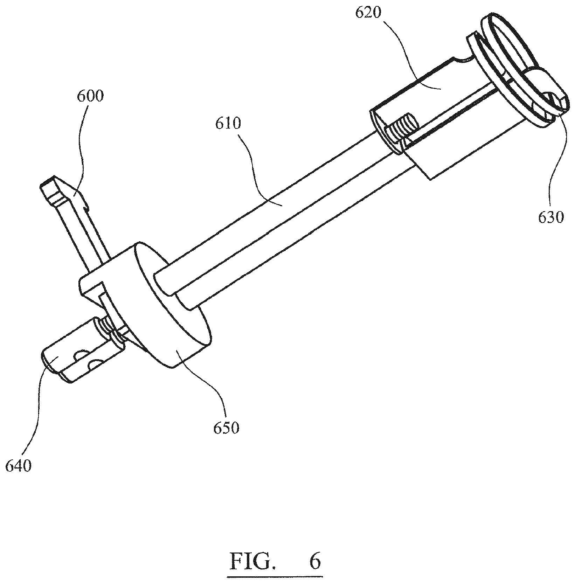

Figures 2 and 3 show the heater arrangement of Figure 1 is more detail. The heater 140 is positioned and configured to be received in a cavity within the aerosol-forming substrate. The cavity includes an air inlet 170. The aerosol-forming substrate 180 is formed in a tubular shape and defines an internal bore having a longitudinal axis. A flow sensor is also provided to detect the air flow through the device. The heater 140 comprises a substantially ring shaped or circular heating element 200 mounted on a support 210. The heating element 200 in this example has a helical shape. A positioning mechanism is configured to move the heating element longitudinally in the direction of arrow A. This may be achieved by moving the heating element 200 alone or by moving the support column 210. A more detailed description of one particular arrangement is provided below with reference to Figure 6.

The positioning mechanism includes an engagement mechanism for moving the heating element towards and away from the aerosol-forming substrate during a positioning process. Figure 3 is a cross-sectional view through the heating element of Figure 2 and illustrates the engagement mechanism. One end of the heating element 200 is connected to the support 210 by a radial arm 220 and the other end of the heating element by a radial arm 230. Rotation of the two radial arms 220 and 230 relative to one another changes the radius of curvature of the heating element 200, so that the heating element can be radially expanded and contracted. In a first, expanded position, the heating element is in contact with the aerosol-forming substrate along substantially its entire length. In a second, retracted position the heating element 200 is spaced from the aerosol-forming substrate, allowing it to be more easily moved in the longitudinal direction. The relative rotation of the radial arms may be achieved by rotating one arm and keeping the other fixed relative to the housing, or by rotating both arms, simultaneously or sequentially.

Electrical power is supplied to the heating element 200 through the support 210. The support itself may be formed fully or partially from conductive material, or a separate conductive path may be provided on or within the support 210.

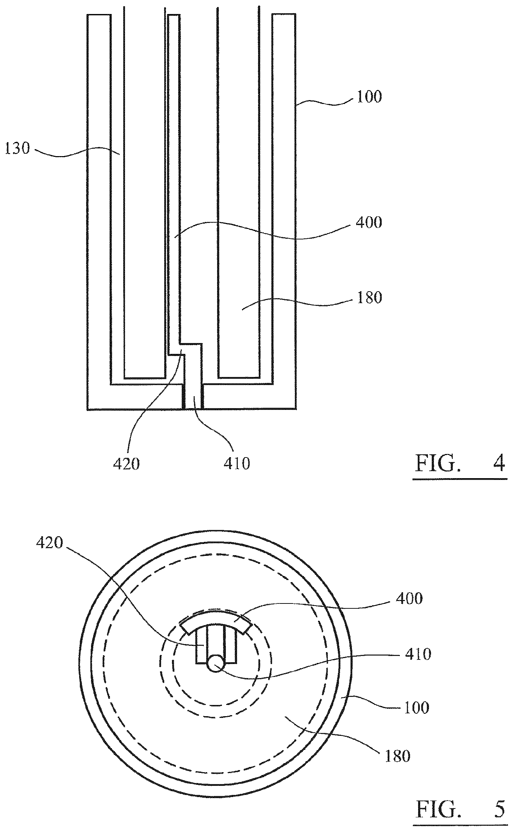

Figure 4 is a schematic illustration of an alternative heater arrangement and positioning mechanism in accordance with the invention. The heater of Figure 4 comprises an elongate heating element 400 mounted on support 410 and radial arms 420. The heating element 400 is configured to heat a section of the aerosol-forming substrate along substantially the entire length of the bore. The heating element is rotated to heat different sections of the aerosol-forming substrate by the positioning mechanism, which comprises support 410 and a stepper motor (not shown) for rotating the support.

The heating element 400 in Figure 4 is shown in contact with the aerosol-forming substrate 180. The heating element may be retracted by an engagement mechanism configured to move the arms 420 away from the aerosol-forming substrate using an electromagnetic actuator.

Electrical power is supplied to the heating element 400 through the support 410. The support itself may be formed fully or partially from conductive material, or a separate conductive path may be provided on or within the support 410.

Figure 5 is an end view of the heater arrangement of Figure 4.

Figure 6 shows an engagement mechanism for expanding and contracting a heating element of the type shown in Figures 2 and 3. The engagement mechanism includes a push button 600. Depression of the button 600 applies a rotation to support rods 610. The engagement mechanism is configured such that it applies rotation to one rod in an opposite sense to the other rod. The rotational movement is transmitted to the torsion bars 620, which are fixed to either end of a helical heating element 630. The torsion bars and support rods are stiffer than the heating element. The rotation of the torsion bars increases the angular distance traveled by the heating element and so reduces the diameter of the helical heating element. With the button 600 depressed the heating element 630 is therefore spaced apart from the aerosol-forming substrate and can be moved in a longitudinal direction to a new position without frictional interference with the aerosol-forming substrate.

When the button is released, the resilient heating element 630 springs back to its original shape and so once again contacts the aerosol-forming substrate. Additional biasing means may be provided within the button mechanism if required. Electrical contacts 640 are spring biased into contact with the support rods 610 so that electrical contact is maintained while allowing the support rods to rotate.

The heating element is moved longitudinally using a manually operated longitudinal positioning mechanism. The whole assembly shown in Figure 6 may be moved within the housing of the device. Flexible wiring may be used to provide the electrical contact between contacts 640 and electrical contacts fixed to the housing (not shown) so that longitudinal movement of the assembly can be accommodated while still allowing for a supply of electrical power. The longitudinal movement of the assembly may be a simple sliding movement of the support element 650 along an internal bore of the housing. A resilient arm or protrusion may be provided on the support element for engagement with features on the internal surface of the bore to provide a clicking noise as they pass one another. The features on the internal surface of the bore may be spaced apart such that a single clicking noise informs the user that the heating element is adjacent a new, unheated section of the aerosol-forming substrate.

More complex mechanisms for longitudinal movement of the heating element are of course possible including automatic mechanisms that move the heating element longitudinally when button 600 is depressed or following each activation of the heating element or sensed user inhalation. Such automatic mechanisms may be powered by a permanent magnet and solenoid or by a stepper motor for example.

It is also possible to include a position detection mechanism together with a manually operated positioning mechanism in order to prevent heating of the same portion of the aerosol-forming substrate twice. For example, an optical sensor may be incorporated into the assembly or housing to determine the position of the heating element. The microcontroller may then disable the supply of power to the heating element or issue a visible or audible alarm if it is determined to be in a position in which it has already been activated for the current aerosol-forming substrate.

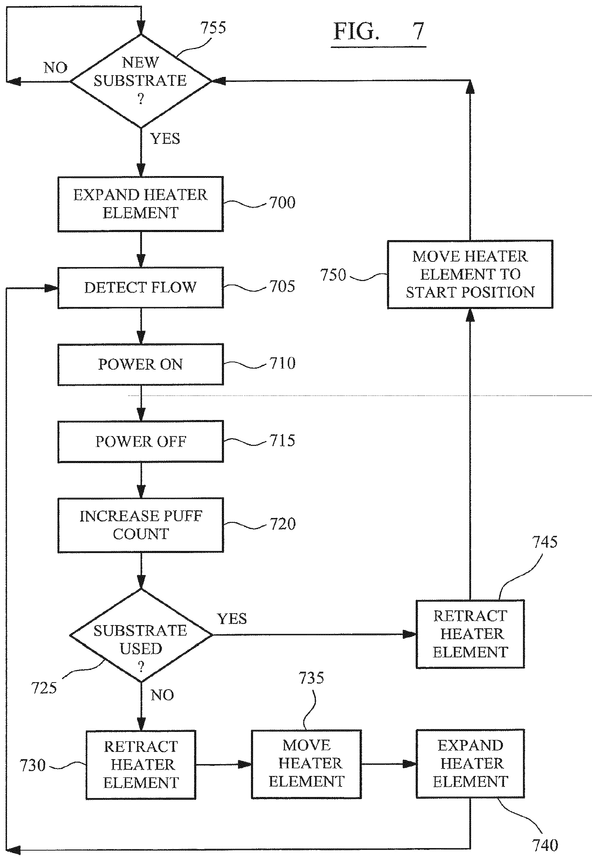

Figure 7 is a flow diagram illustrating an example of the operation of a heater in accordance with Figures 2, 3 and 6. When a new aerosol-forming substrate is to be inserted into the socket, the heating element is in a contracted configuration. In the system shown in Figure 6, this means that the button 600 is pressed. In step 700, once a new aerosol-forming substrate has been inserted, the heating element is expanded by the engagement mechanism to contact the interior surface of the aerosol-forming substrate. The heating element is positioned as close to the filter end of the aerosol-forming substrate as possible. This minimizes the time to first puff for the user. In step 705, a flow of air through the device is detected by the flow sensor, indicating that a user is taking a puff. Following detection of air flow, the microcontroller supplied power to the heating element in step 710. After a fixed duration or fixed amount of power, the microcontroller switches off the power to the heating element, in step 715.

In step 720, a puff count for the aerosol-forming substrate is incremented. The puff count is used to ensure that only a fixed number of heating cycles are used on each aerosol-forming substrate so that no section of the aerosol-forming substrate is heated twice. The puff count increase can be carried out before, simultaneous with or after the supply of power to the heating element.

In step 725, the microcontroller checks the puff count to determine if the aerosol-forming substrate needs to be replaced. If not, the heating element is moved to a new portion of the aerosol-forming substrate by the positioning mechanism. First, in step 730, the heating element is contracted by pressing the button 600 (or by automatic means). The heating element is then moved longitudinally to new position in step 735, as described with reference to Figure 7. The new position may be directly adjacent the previous position or may be distant from it. In this example, the heating element is simply moved to an adjacent position, one step further from the filter end of the aerosol-forming substrate. Once in the desired longitudinal position, the button 600 is released and the heating element expands to contact the new portion of the aerosol-forming substrate in step 740. The heating element is then ready to supply the next puff and the process then returns to step 705.

If in step 725 the microcontroller determines that all available areas of the aerosol-forming substrate have been used, then the aerosol-forming substrate must be replaced. A visual or audible indication may be provided to the user. In order to replace the aerosol-forming substrate, the button 600 is pressed to contract the heating element in step 745. The aerosol-forming substrate can then be easily slid out of the device. While the heating element is contracted, it is moved longitudinally to the start position closest to the filter in step 750. To insert a new aerosol-forming substrate, the button remains pressed, or is pressed again if it has been released, to contract the heating element. In step 755, if an automated mechanism is used, the heating element can be held in a contracted state until a new aerosol-forming substrate is detected. Once a new aerosol-forming substrate is detected the process begins again at step 700.

## Claims

1. An aerosol generating device, comprising :
a housing (100) configured to receive an aerosol-forming substrate (180) having an internal cavity;
a heating element (200; 400), the heating element (200; 400) configured to be received within the internal cavity of the aerosol-forming substrate (180); and
a positioning mechanism coupled to the heating element (200; 400), the positioning mechanism configured to move the heating element (200; 400) between a plurality of positions within the internal cavity.

2. A device according to claim 1, wherein the positioning mechanism further comprises an engagement mechanism, the engagement mechanism configured to move the heating element (200; 400) towards and away from an interior surface of the internal cavity.

3. A device according to claim 2, wherein the engagement mechanism is configured to move the heating element (200; 400) into and out of contact with the interior surface of the aerosol forming substrate (180).

4. A device according to claim 1, 2 or 3, wherein the device is configured to receive a aerosol-forming substrate (180) that is tubular such that the internal cavity is a bore having a longitudinal axis, and wherein the positioning mechanism is configured to move the heating element (200; 400) in a longitudinal direction.

5. A device according to any preceding claim, wherein the heating element (200) is substantially ring shaped or circular.

6. A device according to claim 5 when dependent on claim 2, wherein the heating element (200) is resilient and wherein the engagement mechanism is attached to at least one end of the heating element (200), and is configured to move that end of the heating element (200) in a circumferential direction to radially expand or contract the heating element (200).

7. A device in accordance with any preceding claim, further comprising a microcontroller, the microcontroller configured to control the supply of electrical power to the heating element (200; 400) and configured to activate the positioning mechanism following the supply of predetermined amount or duration of electrical power to the heating element (200; 400).

8. A device in accordance with any preceding claim, wherein the positioning mechanism is configured to conduct electricity to the heating element (200; 400).

## Patentansprüche

1. Aerosolerzeugungsvorrichtung, aufweisend:
ein Gehäuse (100), das ausgelegt ist, ein aerosolbildendes Substrat (180) mit einem inneren Hohlraum aufzunehmen;
ein Heizelement (200; 400), wobei das Heizelement (200; 400) ausgelegt ist, innerhalb des inneren Hohlraums des aerosolbildenden Substrats (180) aufgenommen zu werden; und
einen Positioniermechanismus, der mit dem Heizelement (200; 400) gekoppelt ist, wobei der Positioniermechanismus ausgelegt ist, das Heizelement (200; 400) zwischen mehreren Positionen innerhalb des inneren Hohlraums zu bewegen.

2. Vorrichtung nach Anspruch 1, wobei der Positioniermechanismus weiter einen Eingriffsmechanismus aufweist und der Eingriffsmechanismus ausgelegt ist, das Heizelement (200; 400) zu einer Innenfläche des inneren Hohlraums und davon weg zu bewegen.

3. Vorrichtung nach Anspruch 2, wobei der Eingriffsmechanismus ausgelegt ist, das Heizelement (200; 400) in und aus dem Kontakt mit der Innenfläche des aerosolbildenden Substrats (180) zu bewegen.

4. Vorrichtung nach Anspruch 1, 2 oder 3, wobei die Vorrichtung ausgelegt ist, ein aerosolbildendes Substrat (180) aufzunehmen, das rohrförmig ist, sodass der innere Hohlraum eine Bohrung mit einer Längsachse ist, und wobei der Positioniermechanismus ausgelegt ist, das Heizelement (200; 400) in einer Längsrichtung zu bewegen.

5. Vorrichtung nach einem der vorstehenden Ansprüche, wobei das Heizelement (200) im Wesentlichen ringförmig oder kreisförmig ist.

6. Vorrichtung nach Anspruch 5 wenn abhängig von Anspruch 2, wobei das Heizelement (200) elastisch ist, und wobei der Eingriffsmechanismus an mindestens einem Ende des Heizelements (200) befestigt und ausgelegt ist, dieses Ende des Heizelements (200) in einer Umfangsrichtung zu bewegen, um das Heizelement (200) radial auszudehnen oder zu kontrahieren.

7. Vorrichtung nach einem vorstehenden Anspruch, weiter aufweisend einen Mikrocontroller, wobei der Mikrocontroller ausgelegt ist, das Bereitstellen von elektrischem Strom an das Heizelement (200; 400) zu steuern, und ausgelegt ist, den Positioniermechanismus nach dem Bereitstellen der vorbestimmten Menge oder Zeitdauer an elektrischem Strom an das Heizelement (200; 400) zu aktivieren.

8. Vorrichtung nach einem vorstehenden Anspruch, wobei der Positioniermechanismus ausgelegt ist, Elektrizität zum Heizelement (200; 400) zu leiten.

## Revendications

1. Dispositif de génération d'aérosol comprenant :
un logement (100) configuré pour recevoir un substrat formant aérosol (180) ayant une cavité interne ;
un élément de chauffage (200 ; 400), l'élément de chauffage (200 ; 400) étant configuré pour être reçu dans la cavité interne du substrat formant aérosol (180) ; et
un mécanisme de positionnement accouplé à l'élément de chauffage (200 ; 400), le mécanisme de positionnement étant configuré pour déplacer l'élément de chauffage (200 ; 400) entre une pluralité de positions dans la cavité interne.

2. Dispositif selon la revendication 1, dans lequel le mécanisme de positionnement comprend en outre un mécanisme d'engagement, le mécanisme d'engagement étant configuré pour déplacer l'élément de chauffage (200 ; 400) en direction et à partir d'une surface intérieure de la cavité interne.

3. Dispositif selon la revendication 2, dans lequel le mécanisme d'engagement est configuré pour déplacer l'élément de chauffage (200 ; 400) en contact et hors contact avec la surface intérieure du substrat formant aérosol (180).

4. Dispositif selon la revendication 1, 2 ou 3, dans lequel le dispositif est configuré pour recevoir un substrat formant aérosol (180) qui est tubulaire, de sorte que la cavité interne est un alésage ayant un axe longitudinal, et où le mécanisme de positionnement est configuré pour déplacer l'élément de chauffage (200 ; 400) dans une direction longitudinale.

5. Dispositif selon une quelconque revendication précédente, dans lequel l'élément de chauffage (200) est substantiellement de forme annulaire ou circulaire.

6. Dispositif selon la revendication 5 lorsqu'il est dépendent de la revendication 2, dans lequel l'élément de chauffage (200) est résilient et dans lequel le mécanisme d'engagement est attaché à au moins une extrémité de l'élément de chauffage (200), et est configuré pour déplacer cette extrémité de l'élément de chauffage (200) dans une direction circonférentielle pour étendre ou contracter radialement l'élément de chauffage (200).

7. Dispositif selon une quelconque revendication précédente, comprenant en outre un microcontrôleur, le microcontrôleur étant configuré pour commander l'alimentation de énergie électrique vers l'élément de chauffage (200 ; 400) et étant configuré pour activer le mécanisme de positionnement suite à l'alimentation d'une quantité ou d'une durée prédéterminée de énergie électrique vers l'élément de chauffage (200 ; 400).

8. Dispositif selon une quelconque revendication précédente, dans lequel le mécanisme de positionnement est configuré pour conduire de l'électricité vers l'élément de chauffage (200 ; 400).
